# EUROPEAN PATENT APPLICATION

(11) **EP 4 049 658 A1**
(43) Date of publication of application: **31.08.2022**
(21) Application number: 20878390.2
(22) Date of filing: 23.10.2020
(51) Int. Cl.: A61K 31/4748, A61K 45/00, A61P 7/00

(54) **THERAPEUTIC OR PROPHYLACTIC AGENT FOR CACHEXIA**

(30) Priority: 24.10.2019 JP 2019193743
(71) Applicant: Toray Industries, Inc., Tokyo, 103-8666 (JP)
(72) Inventor: OMORI Yu, Kamakura-shi, Kanagawa 248-8555 (JP); UCHIDA Masashi, Kamakura-shi, Kanagawa 248-8555 (JP)
(74) Representative: Kador & Partner PartG mbB
(86) International application number: PCT/JP2020/039870
(87) International publication number: WO 2021/079978

(57) **Abstract**

The present invention addresses the problem of providing a therapeutic or prophylactic agent that is for cachexia and that exhibits significant efficacy in therapy of cachexia with few side effects. The present invention provides a therapeutic or prophylactic agent that is for cachexia and that contains a combination of a ghrelin receptor agonist a compound having a morphinan skeleton, represented by the following compounds, or a pharmacologically acceptable acid addition salt thereof.

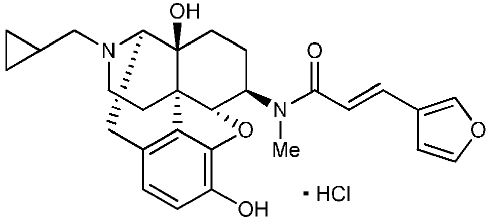

## Description

### TECHNICAL FIELD

The present invention relates to a therapeutic or prophylactic agent for cachexia.

### BACKGROUND ART

Cachexia is a complex syndrome of metabolic abnormality caused in association with an underlying disease, and is defined as muscle loss irrespective of fat loss. Examples of the underlying disease which causes cachexia include chronic diseases such as malignant tumors, tuberculosis, diabetes, blood diseases, endocrine diseases, infectious diseases, and acquired immunodeficiency syndrome. Cachexia is known as a systemic syndrome having prominent symptoms such as remarkable weight loss, anemia, edema, anorexia, general debility, and malaise (Non Patent Literature 1).

Among these, the cachexia derived from malignant tumors as the underlying disease is called cancer cachexia, and it is said that the cachexia constitutes about 20% of death causes of malignant tumors.

In the cancer cachexia, patients significantly lose their physical strength with ongoing symptoms. For this reason, not only treatments with antitumor drugs cannot be continued, but also their reactivity to those antitumor drugs is reduced. Nutritional supplementation to improve the cachectic symptoms conversely accelerates exacerbation of malignant tumors, and thus, the onset of the cancer cachexia is a large obstacle to treatment of malignant tumors. It is reported that even if patients have physical strength to allow administration of antitumor drugs, side effects due to antitumor drugs, such as bone marrow toxicity, are caused and no improvement in cachexia is observed (Non Patent Literature 2).

There has been no standard and effective treatment method for cachexia so far. As substances having cachexia improving action, a ghrelin receptor agonist (Patent Literature 1), an androgen receptor modulator (Patent Literature 2), and the like are known.

Ghrelin is a peptide hormone discovered as an endogenous ligand (GHSR agonist) for a growth hormone secretagogue receptor (GHSR). Ghrelin is produced mainly in stomachs through a ghrelin receptor in human and animals to enhance growth hormone secretion (Non Patent Literature 3). Besides, it is reported that ghrelin has a variety of actions such as increase of food intake (improvement of eating disorder), enhancement of gastric acid secretion and gastric peristalsis, and increase of skeletal muscle (Non Patent Literature 3). Anamorelin, which is a ghrelin receptor agonist, significantly increases lean body mass in third phase clinical trials, and is expected as a cachexia therapeutic agent (Patent Literature 3).

However, an increase in ghrelin concentration in blood is recognized in cancer cachexia patients, and a reduction in ghrelin reactivity, namely, ghrelin resistance is caused (Non Patent Literature 4). Rikkunshito, a traditional Japanese herbal medicine, partially improves the ghrelin resistance, which suggests a therapeutic potential to cancer cachexia (Non Patent Literature 5).

On the other hand, a compound having a morphinan skeleton or a pharmacologically acceptable acid addition salt thereof having opioid κ receptor agonistic properties and applications thereof as a pain reliever and a diuretic agent are disclosed (Patent Literature 4). Applications thereof as a cachexia therapeutic agent (Patent Literature 5) are also disclosed.

### CITATION LIST

### PATENT LITERATURE

Patent Literature 1: International Publication No. WO 05/097261
Patent Literature 2: International Publication No. WO 02/066475
Patent Literature 3: International Publication No. WO 16/036598
Patent Literature 4: International Publication No. WO 93/015081
Patent Literature 5: International Publication No. WO 12/105475

### NON PATENT LITERATURE

Non Patent Literature 1: Kern et al., Journal of Parenteral and Enteral Nutrition, 1988, Vol. 12, p. 286-298
Non Patent Literature 2: Nelson et al., Journal of Clinical Oncology, 1994, Vol. 12, p. 213-225
Non Patent Literature 3: Kojima, Kangawa, Physiological Reviews, 2005, Vol. 85, p. 495-522
Non Patent Literature 4: Nakazato et al., Nature, 2001, Vol. 409, p. 194-198
Non Patent Literature 5: Terawaki et al., PLos One, 2017, Vol. 12, p. e0173113

### SUMMARY OF INVENTION

### TECHNICAL PROBLEM

However, any improving effect on metabolic abnormality is not observed in appetite stimulants used in treatment of cachexia, such as ghrelin receptor agonists, and therapeutic effects of the appetite stimulants are restricted due to ghrelin resistance of patients. Steroidal anti-inflammatory drugs are difficult to use for a long time due to their strong side effects.

For compounds having a morphinan skeleton or pharmacologically acceptable acid addition salts thereof, there is no report about a reduction in dosage, an increase in cachexia therapeutic effect and a reduction in side effects of them in combined use with a ghrelin receptor agonist. The therapeutic effect to cachexia in ghrelin-resistant states is not suggested. Thus, in treatments of cachexia at present, creation of a medical drug based on a new mechanism has been expected.

Accordingly, an object of the present invention is to provide a therapeutic or prophylactic agent for cachexia which exhibits remarkable drug efficacy in treatments of cachexia with reduced side effects.

### SOLUTION TO PROBLEM

The present inventors, who have conducted extensive research to solve the above problems, have found that a high therapeutic or prophylactic effect to cachexia is provided by use of a specific compound having a morphinan skeleton or a pharmacologically acceptable acid addition salt thereof and a ghrelin receptor agonist in combination, and have completed the present invention.

Thus, the present invention provides a therapeutic or prophylactic agent for cachexia wherein a compound represented by General Formula (I) below or a pharmacologically acceptable acid addition salt thereof and a ghrelin receptor agonist are used in combination. 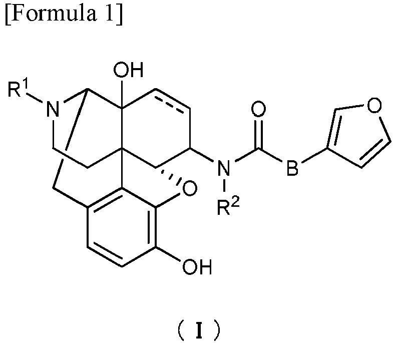 Herein, a double line composed of a dotted line and a solid line represents a double bond or a single bond, R¹ represents a cycloalkylalkyl having 4 to 7 carbon atoms, R² represents a linear or branched alkyl having 1 to 5 carbon atoms, and B represents a -CH=CH-.

As one aspect according to the therapeutic or prophylactic agent for cachexia, the present invention also provides a therapeutic or prophylactic agent for cachexia comprising the compound represented by General Formula (I) or a pharmacologically acceptable acid addition salt thereof and a ghrelin receptor agonist as active ingredients.

In the compound represented by General Formula (I), preferably, R¹ is a cyclopropylmethyl, a cyclobutylmethyl, a cyclopentylmethyl, or a cyclohexylmethyl, and R² is a methyl, an ethyl, or a propyl; more preferably, R¹ is a cyclopropylmethyl, R² is a methyl, and B is a trans-form -CH=CH-.

The compound represented by General Formula (I) is more preferably (-)-17-(cyclopropylmethyl)-3,14β-dihydroxy-4,5α-epoxy-6β-[N-methyl-trans-3-(3-furyl)acrylamide] morphinan exemplified below, and the ghrelin receptor agonist is more preferably anamorelin or a pharmacologically acceptable salt thereof.

The therapeutic or prophylactic agent for cachexia is preferably a therapeutic or prophylactic agent for cancer cachexia, and can also be suitably used as a therapeutic or prophylactic agent for cancer cachexia in ghrelin-resistant states.

As one embodiment according to the therapeutic or prophylactic agent for cachexia, the present invention provides a pharmaceutical composition for treating or preventing cachexia wherein a compound represented by General Formula (I) or a pharmacologically acceptable acid addition salt thereof and a ghrelin receptor agonist are used in combination, and which comprises one or more pharmaceutically acceptable carriers.

As one embodiment according to the therapeutic or prophylactic agent for cachexia, the present invention provides a pharmaceutical combination or a kit for treating or preventing cachexia, wherein a compound represented by General Formula (I) or a pharmacologically acceptable acid addition salt thereof and a ghrelin receptor agonist are used in combination.

As one embodiment according to the therapeutic or prophylactic agent for cachexia, the present invention provides use of a combination of a compound represented by General Formula (I) or a pharmacologically acceptable acid addition salt thereof and a ghrelin receptor agonist in the manufacture of a medicament for treating or preventing cachexia (such as the therapeutic or prophylactic agent for cachexia). In addition, as one embodiment according to the therapeutic or prophylactic agent for cachexia, the present invention provides a combination for treating or preventing cachexia of a compound represented by General Formula (I) or a pharmacologically acceptable acid addition salt thereof and a ghrelin receptor agonist.

As one embodiment according to the therapeutic or prophylactic agent for cachexia, the present invention provides a therapeutic or prophylactic agent for cachexia comprising a compound represented by General Formula (I) or a pharmacologically acceptable acid addition salt thereof as an active ingredient, which is used in combination (as a combined use) with the ghrelin receptor agonist, and also provides use of a compound represented by General Formula (I) or a pharmacologically acceptable acid addition salt thereof in the manufacture of a medicament for treating or preventing cachexia (such as the therapeutic or prophylactic agent for cachexia), which is used in combination (as a combined use) with the ghrelin receptor agonist.

In addition, as one embodiment according to the therapeutic or prophylactic agent for cachexia, the present invention provides a therapeutic or prophylactic agent for cachexia comprising a ghrelin receptor agonist as an active ingredient, which is used in combination (as a combined use) with the compound represented by General Formula (I) or a pharmacologically acceptable acid addition salt thereof, and also provides use of a ghrelin receptor agonist in the manufacture of a medicament for treating or preventing cachexia (such as the therapeutic or prophylactic agent for cachexia), which is used in combination (as a combined use) with the compound represented by General Formula (I) or a pharmacologically acceptable acid addition salt thereof.

In addition, as one embodiment according to the therapeutic or prophylactic agent for cachexia, the present invention provides a compound represented by General Formula (I) or a pharmacologically acceptable acid addition salt thereof for use in treatment or prevention of cachexia, which is used in combination (as a combined use) with the ghrelin receptor agonist, and also provides a ghrelin receptor agonist for use in treatment or prevention of cachexia, which is used in combination (as a combined use) with the compound represented by General Formula (I) or a pharmacologically acceptable acid addition salt thereof.

Furthermore, as one embodiment according to the therapeutic or prophylactic agent for cachexia, the present invention provides a method for treating or preventing cachexia, which comprises administering a compound represented by General Formula (I) or a pharmacologically acceptable acid addition salt thereof and a ghrelin receptor agonist in combination (as a combined use) to a patient in need of treatment or prevention of cachexia.

### ADVANTAGEOUS EFFECTS OF INVENTION

The therapeutic or prophylactic agent for cachexia according to the present invention can ensure a reduction in dosage(s) of the compound having a morphinan skeleton and/or the ghrelin receptor agonist, a reduction in concerns about side effects, and can improve systemic syndromes having main symptoms such as remarkable weight loss, anemia, edema, anorexia, general debility, and malaise in a patient having cachexia. Furthermore, the therapeutic or prophylactic agent for cachexia according to the present invention can also improve symptoms of cachexia in ghrelin-resistant states.

### BRIEF DESCRIPTION OF DRAWINGS

[Figure 1] Figure 1 is a graph showing the increasing action of cumulative food intake by administration of Compound 1 and anamorelin in combination in a tumor-bearing mouse model (non-small cell lung cancer model having ghrelin resistance).
[Figure 2] Figure 2 is a graph showing the increasing action of body weight by administration of Compound 1 and anamorelin in combination in a tumor-bearing mouse model (non-small cell lung cancer model having ghrelin resistance).
[Figure 3] Figure 3 is a graph showing the increasing action of cumulative food intake by administration of Compound 1 and anamorelin in combination in a tumor-bearing mouse model (non-small cell lung cancer model).
[Figure 4] Figure 4 is a graph showing the increasing action of body weight by administration of Compound 1 and anamorelin in combination in a tumor-bearing mouse model (non-small cell lung cancer model).
[Figure 5] Figure 5 is a graph showing the increasing action of cumulative food intake by administration of Compound 1 and anamorelin in combination in a tumor-bearing mouse model (skin cancer model).
[Figure 6] Figure 6 is a graph showing the increasing action of body weight by administration of Compound 1 and anamorelin in combination in a tumor-bearing mouse model (skin cancer model).

This specification encompasses the contents of the description and/or the drawings described in Japanese Patent Application No. 2019-193743, on which the priority of this application is based.

### DESCRIPTION OF EMBODIMENTS

The present invention is characterized by being a therapeutic or prophylactic agent for cachexia wherein a compound represented by General Formula (II) below or a pharmacologically acceptable acid addition salt thereof and a ghrelin receptor agonist are used in combination. 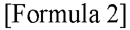 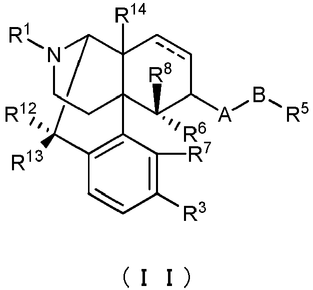 Herein, a double line composed of a dotted line and a solid line represents a double bond or a single bond,
R¹ represents an alkyl having 1 to 5 carbon atoms, a cycloalkylalkyl having 4 to 7 carbon atoms, a cycloalkenylalkyl having 5 to 7 carbon atoms, an aryl having 6 to 12 carbon atoms, an aralkyl having 7 to 13 carbon atoms, an alkenyl having 4 to 7 carbon atoms, an allyl, a furan-2-ylalkyl where the alkyl moiety has 1 to 5 carbon atoms, or a thiophen-2-ylalkyl where the alkyl moiety has 1 to 5 carbon atoms,
R¹⁴ represents a hydrogen, a hydroxy, a nitro, an alkanoyloxy having 1 to 5 carbon atoms, an alkoxy having 1 to 5 carbon atoms, an alkyl having 1 to 5 carbon atoms or a NR⁹R¹⁰ wherein R⁹ represents a hydrogen or an alkyl having 1 to 5 carbon atoms, and R¹⁰ represents a hydrogen, an alkyl having 1 to 5 carbon atoms, or a -(C=O)R¹¹ where R¹¹ represents a hydrogen, a phenyl, or an alkyl having 1 to 5 carbon atoms,
R³ represents a hydrogen, a hydroxy, an alkanoyloxy having 1 to 5 carbon atoms, or an alkoxy having 1 to 5 carbon atoms,
A represents a -XC(=Y)-, a -XC(=Y)Z-, a -X-, or a -XSO₂- wherein X, Y, and Z each independently represent a NR⁴ where R⁴ represents a hydrogen, a linear or branched alkyl having 1 to 5 carbon atoms, or an aryl having 6 to 12 carbon atoms; and when two or more R⁴s are present, these may be the same or different, a S, or an O,
B represents a valence bond, a linear or branched alkylene having 1 to 14 carbon atoms where the alkylene may be substituted by at least one or more substituents selected from the group consisting of an alkoxy having 1 to 5 carbon atoms, an alkanoyloxy having 1 to 5 carbon atoms, a hydroxy, a fluorine, a chlorine, a bromine, an iodine, an amino, a nitro, a cyano, a trifluoromethyl, and a phenoxy, and 1 to 3 methylenes thereof may be substituted by carbonyls, a linear or branched non-cyclic unsaturated hydrocarbon having 2 to 14 carbon atoms and containing 1 to 3 double bonds and/or triple bonds where the non-cyclic unsaturated hydrocarbon may be substituted by at least one or more substituents selected from the group consisting of an alkoxy having 1 to 5 carbon atoms, an alkanoyloxy having 1 to 5 carbon atoms, a hydroxy, a fluorine, a chlorine, a bromine, an iodine, an amino, a nitro, a cyano, a trifluoromethyl, and a phenoxy, and 1 to 3 methylenes thereof may be substituted by carbonyls, a linear or branched saturated or unsaturated hydrocarbon having 1 to 14 carbon atoms and containing 1 to 5 thioether bonds, ether bonds, and/or amino bonds where the heteroatoms are not directly bound to A, and 1 to 3 methylenes of the hydrocarbon may be substituted by carbonyls, a linear or branched non-cyclic unsaturated hydrocarbon having 2 to 14 carbon atoms and containing 1 to 3 double bonds and/or triple bonds where the hydrocarbon may be substituted by at least one or more substituents selected from the group consisting of an alkoxy having 1 to 5 carbon atoms, an alkanoyloxy having 1 to 5 carbon atoms, a hydroxy, a fluorine, a chlorine, a bromine, an iodine, an amino, a nitro, a cyano, a trifluoromethyl, and a phenoxy, and 1 to 3 methylenes thereof may be substituted by carbonyls, or a linear or branched saturated or unsaturated hydrocarbon having 1 to 14 carbon atoms and containing 1 to 5 thioether bonds, ether bonds, and/or amino bonds where the heteroatoms are not directly bound to A, and 1 to 3 methylenes of the hydrocarbon may be substituted by carbonyls),
R⁵ represents a hydrogen or an organic group having any of the following basic skeletons: 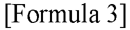 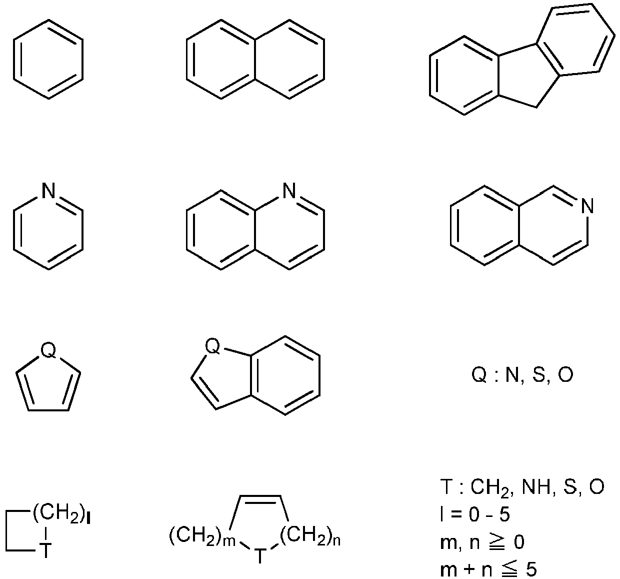 wherein Q represents an N, an O, or a S, T represents a CH₂, an NH, a S, or an O, l represents an integer of 0 to 5, m and n each independently represent an integer of 0 to 5, the total of m and n is 5 or less, and each of the organic groups may be substituted by at least one or more substituents selected from the group consisting of an alkyl having 1 to 5 carbon atoms, an alkoxy having 1 to 5 carbon atoms, an alkanoyloxy having 1 to 5 carbon atoms, a hydroxy, a fluorine, a chlorine, a bromine, an iodine, an amino, a nitro, a cyano, an isothiocyanato, a trifluoromethyl, a trifluoromethoxy, and a methylenedioxy,
R⁶ represents a hydrogen, and
R⁷ represents a hydrogen, a hydroxy, an alkoxy having 1 to 5 carbon atoms, or an alkanoyloxy having 1 to 5 carbon atoms, or
R⁶ and R⁷ together represent a -O-, a -CH₂-, or a -S-,
R⁸ represents a hydrogen, an alkyl having 1 to 5 carbon atoms, or an alkanoyl having 1 to 5 carbon atoms,
R¹² and R¹³ together represent a hydrogen, one of them represents a hydrogen and the other represents a hydroxy, or these together represent an oxo, and
General Formula (II) includes a (+) form, a (-) form, and a ± form.

In the therapeutic or prophylactic agent for cachexia, among these compounds represented by General Formula (II) or pharmacologically acceptable acid addition salts thereof, the compound represented by General Formula (I) having a morphinan skeleton already shown or a pharmacologically acceptable acid addition salt thereof is preferable.

In General Formula (I), the double line composed of a dotted line and a solid line represents a double bond or a single bond, and is preferably a single bond.

In General Formula (I), R¹ represents a cycloalkylalkyl having 4 to 7 carbon atoms. Among these, R¹ is preferably a cyclopropylmethyl, a cyclobutylmethyl, a cyclopentylmethyl, and a cyclohexylmethyl, and particularly preferably a cyclopropylmethyl.

R² represents a linear or branched alkyl having 1 to 5 carbon atoms. Among these, R² is preferably a methyl, an ethyl, and a propyl, and particularly preferably a methyl.

B represents a -CH=CH-. B is preferably a trans-form -CH=CH-.

The compound represented by General Formula (I) is particularly preferably a (-)-form compound where the double line composed of a dotted line and a solid line is a single bond, R¹ is a cyclopropylmethyl, R² is a methyl, and B is a trans-form -CH=CH-, namely, (-)-17-(cyclopropylmethyl)-3,14β-dihydroxy-4,5α-epoxy-6β-[N-methyl-trans-3-(3-furyl)acrylamide] morphinan.

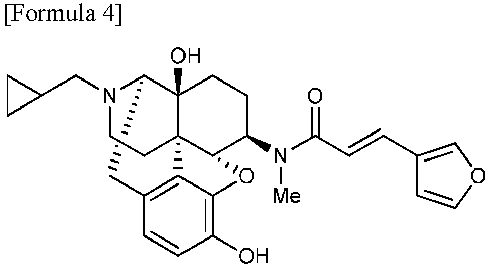

The compound represented by General Formula (I) or a pharmacologically acceptable acid addition salt thereof can be prepared by the method described in WO 93/015081.

Among the compounds represented by General Formula (II), a compound where R¹² and R¹³ both represent a hydrogen can be prepared by the method described in WO 93/015081. In addition, a compound where R¹² and R¹³ together represent an oxo can be prepared by, for example, the method described in an article by Horikiri et al. (Chem. Pharm. Bull., 2004, Vol. 52, No. 6, p. 664-669) and WO 93/015081 using a compound which can be prepared by the method described in an article by Horikiri and Kawamura (Heterocycles, 2004, Vol. 63, p. 865-870) and an article by Sagara et al. (Bioorg. Med. Chem. Lett., 1995, Vol. 5, p. 1505-1508), as a raw material. Furthermore, a compound where R¹² represents a hydroxy and R¹³ represents a hydrogen can be prepared by the method described in the article by Horikiri et al. above.

In the therapeutic or prophylactic agent for cachexia, as the ghrelin receptor agonist known peptide compounds or known non-peptide compounds can be used. Examples of the peptide compound include ghrelin as an endogenous ligand and pralmorelin. Examples of the non-peptide compound include anamorelin, macimorelin, ibutamoren methanesulfonate, ulimorelin, capromorelin, and SM-130686. If the ghrelin receptor agonist is a salt, examples thereof also include dissociated forms thereof; and if the ghrelin receptor agonist can form a salt, examples thereof also include pharmacologically acceptable salts thereof. If the ghrelin receptor agonist is a non-peptide compound, anamorelin or a pharmacologically acceptable salt thereof is preferred.

Among ghrelin receptor agonists, as anamorelin, 2-amino-N-[(1R)-2-[(3R)-3-benzyl-3-(N,N',N'-trimethylhydrazinocarbonyl)piperidin-1-yl-]-1-(1H-indol-3-ylmethyl)-2-oxoethyl]-2-methylpropionamide can be used, which can be prepared by the method described in US 6576659.

In the therapeutic or prophylactic agent for cachexia, a preferred aspect of the compound represented by General Formula (I) or a pharmacologically acceptable acid addition salt thereof and a preferred aspect of the ghrelin receptor agonist can be optionally combined. Examples thereof include a combination of (-)-17-(cyclopropylmethyl)-3,14β-dihydroxy-4,5α-epoxy-6β-[N-methyl-trans-3-(3-furyl)acrylamide]morphinan or a pharmacologically acceptable acid addition salt thereof and anamorelin or a pharmacologically acceptable salt thereof.

The following terms used in this specification are defined as follows, unless otherwise specified.

Examples of the "pharmacologically acceptable acid addition salt" according to the present invention include inorganic acid salts such as a salt of hydrochloride, a sulfate, a nitrate, a hydrobromate, a hydroiodide, and a phosphate; organic carboxylic acid salts such as an acetate, a lactate, a citrate, an oxalate, a glutarate, a malate, a tartrate, a fumarate, a mandelate, a maleate, a benzoate, and a phthalate; and organic sulfonates such as a methanesulfonate, an ethanesulfonate, a benzenesulfonate, a p-toluenesulfonate, and a camphorsulfonate. Among these, a salt of hydrochloride, a hydrobromate, a phosphate, a tartrate, and a methanesulfonate are preferably used.

Examples of the "pharmacologically acceptable salt" according to the present invention include the acid addition salts above, inorganic basic salts such as a sodium salt, a potassium salt, a calcium salt, a magnesium salt, and an ammonium salt; and organic basic salts such as a methylamine salt, a diethylamine salt, a trimethylamine salt, a triethylamine salt, a pyridinium salt, a triethanolamine salt, an ethylenediamine salt, and a guanidine salt.

The "ghrelin receptor" according to the present invention is also represented by GHSR, GHDP, or GHS-R, and means a protein (UniProt ID: Q92847) coded by the gene (Ensembl ID: ENSG00000121853) in human.

The "ghrelin receptor agonist" according to the present invention means a peptide compound or a non-peptide compound which acts on the ghrelin receptor and exhibits the same action as that of ghrelin.

The "cachexia" according to the present invention includes systemic syndromes having main symptoms such as remarkable weight loss, anemia, edema, anorexia, general debility, and malaise in chronic diseases such as malignant tumors, tuberculosis, diabetes, blood diseases, endocrine diseases, infectious diseases, and acquired immunodeficiency syndromes. Examples thereof include cancer cachexia, tuberculous cachexia, diabetic cachexia, blood disease-derived cachexia, endocrine disease-derived cachexia, infectious disease-derived cachexia, and cachexia derived from acquired immunodeficiency syndrome. The therapeutic or prophylactic agent for cachexia is preferably used in cancer cachexia caused by malignant tumors among these types of cachexia.

The "malignant tumor" (also called as cancer or malignant neoplasm) according to the present invention includes epithelial tissue-derived "cancer (also called as carcinoma)", non-epithelial tissue-derived "sarcoma", and those derived from hemopoietic organs. Examples thereof include malignant melanoma, malignant bone tumor, gastric cancer, hepatocyte cancer, acute myeloid leukemia, acute lymphocytic leukemia, uterine cervical cancer, uterine cancer, esophagus cancer, pancreatic cancer, prostate cancer, colorectal cancer, breast cancer, lung cancer, bladder cancer, and ovarian cancer.

The therapeutic or prophylactic agent for cachexia has cachexia improving action, namely, has action to improve systemic syndromes having main symptoms such as remarkable weight loss, anemia, edema, anorexia, general debility, and malaise, which are expressed in chronic diseases such as malignant tumor, tuberculosis, diabetes, blood diseases, endocrine disease, infectious disease, and acquired immunodeficiency syndromes.

The therapeutic or prophylactic agent for cachexia is used as a therapeutic or prophylactic agent for cachexia for mammals (such as human, mice, rats, rabbits, dogs, cats, bovines, horses, pigs, and monkeys).

The therapeutic or prophylactic agent for cachexia wherein the compound represented by General Formula (I) or a pharmacologically acceptable acid addition salt thereof and the ghrelin receptor agonist are used in combination can be orally or parenterally administered as a mixture thereof, namely in the form of a combination drug as it is, or further combined with a pharmaceutically acceptable carrier. Alternatively, the compound represented by General Formula (I) or a pharmacologically acceptable acid addition salt thereof and the ghrelin receptor agonist can be prepared alone, i.e., as single drugs rather than as a combination drug; and these single drugs can be simultaneously administered as they are, or as mixtures with pharmaceutically acceptable additives which are usually used in the drug formulation field, respectively. Furthermore, these single drugs can also be separately administered with an appropriate interval at different timings. In these cases, forms of formulations and administration routes of these single drugs do not need to be the same, and may be different. The "appropriate interval" can be verified clinically or by animal tests.

The therapeutic or prophylactic agent for cachexia comprises a combination of a compound represented by General Formula (I) or a pharmacologically acceptable acid addition salt thereof and a ghrelin receptor agonist, and can optionally comprise one or more pharmaceutically acceptable carriers, and particularly can consist of a combination of a compound represented by General Formula (I) or a pharmacologically acceptable acid addition salt thereof and a ghrelin receptor agonist.

As another aspect according to the present invention, the therapeutic or prophylactic agent for cachexia comprises a compound represented by General Formula (I) or a pharmacologically acceptable acid addition salt thereof and a ghrelin receptor agonist as active ingredients. The therapeutic or prophylactic agent for cachexia comprises a compound represented by General Formula (I) or a pharmacologically acceptable acid addition salt thereof and a ghrelin receptor agonist as active ingredients, and can optionally comprise one or more pharmaceutically acceptable carriers, and particularly can consist of a compound represented by General Formula (I) or a pharmacologically acceptable acid addition salt thereof and a ghrelin receptor agonist.

As one embodiment according to the present invention, a pharmaceutical composition for treating or preventing cachexia is provided, wherein a compound represented by General Formula (I) or a pharmacologically acceptable acid addition salt thereof and a ghrelin receptor agonist are used in combination, and which comprises one or more pharmaceutically acceptable carriers. The pharmaceutical composition according to this embodiment preferably comprises a compound represented by General Formula (I) or a pharmacologically acceptable acid addition salt thereof and a ghrelin receptor agonist as active ingredients, and further comprises one or more pharmaceutically acceptable carriers.

As another embodiment according to the present invention, a pharmaceutical combination for treating or preventing cachexia is provided, wherein a compound represented by General Formula (I) or a pharmacologically acceptable acid addition salt thereof and a ghrelin receptor agonist are used in combination. The pharmaceutical combination according to this embodiment preferably comprises a compound represented by General Formula (I) or a pharmacologically acceptable acid addition salt thereof and a ghrelin receptor agonist in combination. For example, the pharmaceutical combination according to this embodiment may be provided in the form of a single formulation containing the active ingredients above (namely, a combination drug), or may be provided in the form of a plurality of single drugs where the active ingredients above are separately formulated. If the pharmaceutical combination according to this embodiment is in the form of a plurality of single drugs, the pharmaceutical combination according to this embodiment may be provided in the form of a kit comprising a plurality of pharmaceutical formulations comprising each of the active ingredients above, and optionally an instruction for administering the pharmaceutical formulations.

As another embodiment according to the present invention, use of a combination of a compound represented by General Formula (I) or a pharmacologically acceptable acid addition salt thereof and a ghrelin receptor agonist in the manufacture of a medicament for treating or preventing cachexia (such as a therapeutic or prophylactic agent for cachexia) is provided. In addition, as another embodiment according to the present invention, a combination for treating or preventing cachexia of a compound represented by General Formula (I) or a pharmacologically acceptable acid addition salt thereof and a ghrelin receptor agonist is provided.

For the compound represented by General Formula (I) or a pharmacologically acceptable acid addition salt thereof and the ghrelin receptor agonist, the form of formulation for oral administration thereof as single drugs or a combination drug can be selected, for example, from tablets, capsules, oral disintegrants, powders, and granules, and that for parenteral administration can be selected, for example, from intravenous rapid infusion, intravenous continuous infusion, intramuscular infusion, subcutaneous injection, intracutaneous injection, tape agents, and patch agents.

The single drugs of the compound represented by General Formula (I) or a pharmacologically acceptable acid addition salt thereof and the ghrelin receptor agonist or a combination drug thereof in the above form of formulation can be prepared by a known production method usually used in the drug formulation field. In this case, these can be prepared by adding a filler, a binder, a lubricant, a disintegrant, a sweetener, a surfactant, a suspending agent, and an emulsifier which are usually used in the drug formulation field and pharmaceutically acceptable as needed.

Although the content of the compound represented by General Formula (I) or a pharmacologically acceptable acid addition salt thereof in the therapeutic or prophylactic agent for cachexia can be adjusted, not particularly limited, to be usually 0.1 µg to 100 mg per dose. The dosage thereof can be appropriately selected depending on the symptom, age, sex, and weight of the patient or the administration method. Usually, the compound represented by General Formula (I) or a pharmacologically acceptable acid addition salt thereof is given in an amount of 0.1 µg to 20 mg, preferably 1 µg to 10 mg, more preferably 1 µg to 40 µg per day for an adult. These can be each administered in a single dose or in several doses. If these are provided in a kit, single pharmaceutical formulations may be administered at the same time, or may be separately administered with an interval.

Although the content of anamorelin or a pharmacologically acceptable salt thereof in the therapeutic or prophylactic agent for cachexia can be adjusted, not particularly limited, to be usually 10 µg to 10000 mg per dose. The dosage thereof can be appropriately selected depending on the symptom, age, sex, and weight of the patient or the administration method. Usually, anamorelin or a pharmacologically acceptable salt thereof is given in an amount of about 10 µg to about 2000 mg, preferably about 100 µg to about 1000 mg, more preferably about 100 µg to about 400 mg per day for an adult. These can be each administered in a single dose or in several doses. If these are provided in a kit, single pharmaceutical formulations may be administered at the same time, or may be separately administered with an interval.

In the therapeutic or prophylactic agent for cachexia, the combination ratio of the compound represented by General Formula (I) or a pharmacologically acceptable acid addition salt thereof to the ghrelin receptor agonist can be appropriately selected depending on the subject to be administered, the age and weight of the subject to be administered, the symptom, the administration time, the form of formulation, the administration method, and a combination of drugs.

The therapeutic or prophylactic agent for cachexia can be preferably used in a therapeutic or prophylactic agent for cancer cachexia (caused by malignant melanoma, malignant bone tumor, gastric cancer, hepatocyte cancer, acute myeloid leukemia, acute lymphocytic leukemia, uterine cervical cancer, uterine cancer, esophagus cancer, pancreatic cancer, prostate cancer, colorectal cancer, breast cancer, lung cancer, bladder cancer, and ovarian cancer, for example).

The therapeutic or prophylactic agent for cachexia can also be suitably used as a therapeutic or prophylactic agent for cancer cachexia in ghrelin-resistant states.

The therapeutic or prophylactic agent for cachexia, if provided in a kit, can be designed such that a pharmaceutical formulation comprising the compound represented by General Formula (I) or a pharmacologically acceptable acid addition salt thereof formulated by the above method and a pharmaceutical formulation comprising the ghrelin receptor agonist are individually packed, and the pharmaceutical formulations are taken out from the corresponding packages when administered. These pharmaceutical formulations can also be packed in forms suitable for one-time administration in combination thereof.

The therapeutic or prophylactic agent for cachexia can be administered in combination with one or more other drugs used for treating or preventing diseases or reducing or suppressing the symptoms. A drug to be combined may be a low molecular compound, or may be a polymer protein, a polypeptide, an antibody, or a vaccine. At this time, the therapeutic or prophylactic agent for cachexia can be simultaneously administered with the drug to be combined, or can be administered separately therefrom with a time interval. The combination method is suitable as long as these drugs are used in combination, and they may be formed into a combination drug. The dosage of each drug to be combined can be appropriately selected with reference to the dose clinically used. The combination ratio of the therapeutic or prophylactic agent for cachexia to the drug to be combined can be appropriately selected depending on the subject to be administered, the age and weight of the subject to be administered, the symptom, the administration time, the form of formulation, the administration method, and a combination of drugs.

The therapeutic or prophylactic agent for cachexia can be used in combination with a drug such as a chemotherapeutic agent, an immunotherapeutic agent, or a diuretic agent.

Examples of the chemotherapeutic agent include alkylated agents such as cyclophosphamide, ifosfamide, melphalan, busulfane, nimustine, ranimustine, and temozolomide; nucleic acid antimetabolites such as methotrexate, fluorouracil, tegafur, carmofur, doxifluridine, capecitabine, cytarabine, ancitabine, enocitabine, cytarabine ocfosfate, gemcitabine, mercaptopurine, and fludarabine; antitumor antibiotics such as doxorubicin, daunorubicin, pirarubicin, epirubicin, idarubicin, mitoxantrone, mitomycin C, bleomycin, and peplomycin; microtubule inhibitors such as vincristine, vinblastine, vindesine, vinorelbine, paclitaxel, and docetaxel; platinum drugs such as cisplatin, carbplatin, and nedaplatin; topoisomerase inhibitors such as irinotecan, nogitecan, and etoposide; and molecular-target drugs such as trastuzumab, rituximab, and imatinib.

Examples of the immunotherapeutic agent include muramyl dipeptide derivatives, lentinan, schizophyllan, ubenimex, picibanil, Krestin, interferon, interleukin, granulocyte colony-stimulating factors, and erythropoietin.

Examples of the diuretic agent include xanthin derivative drugs such as sodium salicylate and theobromine and calcium salicylate and theobromine; thiazide drugs such as ethiazide, cyclopenthiazide, trichlormethiazide, hydrochlorothiazide, hydroflumethiazide, benzylhydrochlorothiazide, penflutizide, polythiazide, and methyclothiazide; aldosterone antagonists such as spironolactone and triamterene; carbonic anhydrase inhibitors such as acetazolamide; chlorobenzene sulfonamide drugs such as chlorthalidone, mefruside, and indapamide; azosemide, isosorbide, etacrynic acid, piretanide, bumetanide, and furosemide.

Examples of one embodiment according to the therapeutic or prophylactic agent for cachexia include a therapeutic or prophylactic agent for cachexia comprising a compound represented by General Formula (I) or a pharmacologically acceptable acid addition salt thereof as an active ingredient, which is used in combination (as a combined use) with the ghrelin receptor agonist; and a therapeutic or prophylactic agent for cachexia comprising a ghrelin receptor agonist as an active ingredient, which is used in combination (as a combined use) with the compound represented by General Formula (I) or a pharmacologically acceptable acid addition salt thereof.

Examples of one embodiment according to the therapeutic or prophylactic agent for cachexia include use of a compound represented by General Formula (I) or a pharmacologically acceptable acid addition salt thereof in the manufacture of a medicament for treating or preventing cachexia (such as the therapeutic or prophylactic agent for cachexia), which is used in combination (as a combined use) with the ghrelin receptor agonist; and use of a ghrelin receptor agonist in the manufacture of a medicament for treating or preventing cachexia (such as the therapeutic or prophylactic agent for cachexia), which is used in combination (as a combined use) with the compound represented by General Formula (I) or a pharmacologically acceptable acid addition salt thereof.

Examples of one embodiment according to the therapeutic or prophylactic agent for cachexia include a compound represented by General Formula (I) or a pharmacologically acceptable acid addition salt thereof for use in treatment or prevention of cachexia, which is used in combination (as a combined use) with the ghrelin receptor agonist; and a ghrelin receptor agonist for use in treatment or prevention of cachexia, which is used in combination (as a combined use) with the compound represented by General Formula (I) or a pharmacologically acceptable acid addition salt thereof.

Furthermore, examples of one embodiment according to the therapeutic or prophylactic agent for cachexia include a method for treating or preventing cachexia, which comprises administering a compound represented by General Formula (I) or a pharmacologically acceptable acid addition salt thereof and a ghrelin receptor agonist in combination (as a combined use) to a patient in need of treatment or prevention of cachexia. In this embodiment, the compound represented by General Formula (I) or a pharmacologically acceptable acid addition salt thereof and the ghrelin receptor agonist can be administered to the patient in effective amounts (e.g., the contents in the therapeutic or prophylactic agent for cachexia, which are exemplified above). Moreover, in this embodiment, the compound represented by General Formula (I) or a pharmacologically acceptable acid addition salt thereof and the ghrelin receptor agonist can be administered simultaneously, or can be separately administered with an appropriate interval at different timings (e.g., continuously or sequentially with a constant interval).

### EXAMPLES

Hereinafter, the present invention will be specifically described by way of Examples, but these should not be construed as limitations to the present invention.

### (Example 1) Effect of combined use of (-)-17-(cyclopropylmethyl)-3,14β-dihydroxy-4,5α-epoxy-6β-[N-methyl-trans-3-(3-furyl)acrylamide]morphinanhydrochloride (hereinafter, referred to as Compound 1) and anamorelin hydrochloride in tumor-bearing mouse model (non-small cell lung cancer model having ghrelin resistance):

Using tumor-bearing model animals where human alveolar basement epidermal gland cancer cells, A549 cells, were planted into nude mice, examination was performed about drug efficacy on the food intake and the body weight when Compound 1 and anamorelin hydrochloride (MedChemExpress LLC) were combined.

Subculture of A549 cells was performed using a 10% FBS-containing RPMI1640 culture medium. In evaluation of drug efficacy, 7-week old female BALB/C slc/nu/nu mice (Japan SLC, Inc.) were purchased, and were used after habituated for one week. Tumor-bearing model animals were prepared as follows. Specifically, 2.5 × 10⁷ cells of A549 cells were planted to the right abdominal of each mouse by subdermal administration. On Day 41 from the cell plantation, the mice were divided into groups to have an equal averaged tumor volume.

The effects of a variety of compounds on food intake and body weight were examined as follows. Specifically, for 28 days from Day 43 to Day 70 from the cell plantation, the compounds were orally administered daily, and the food intake and the body weight were measured at the same time. For the doses of the compounds, the dose of Compound 1 was 0.25 mg/kg (9 examples), and that of anamorelin hydrochloride was 30 mg/kg (9 examples). In combined administration of Compound 1 and anamorelin hydrochloride, 0.25 mg/kg of Compound 1 and 30 mg/kg of anamorelin hydrochloride were used, where Compound 1 and anamorelin hydrochloride preliminarily dissolved in distilled water were mixed for preparation (9 examples). As a control, distilled water was administered in the same manner (9 examples). The food intake was calculated by subtracting the weight of the lid of each single cage containing food on the previous day from the weight of the lid of the single cage containing food on the day of measurement.

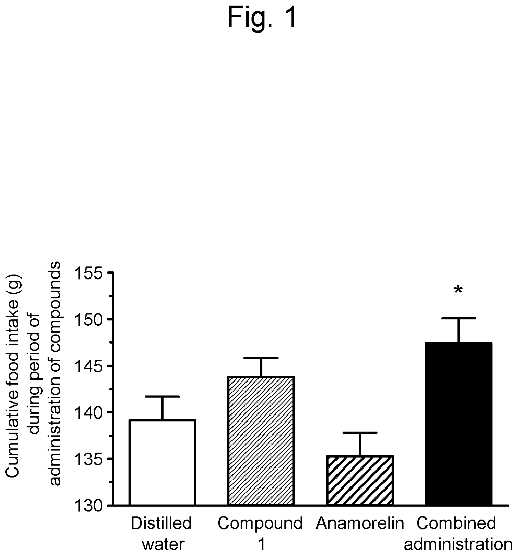

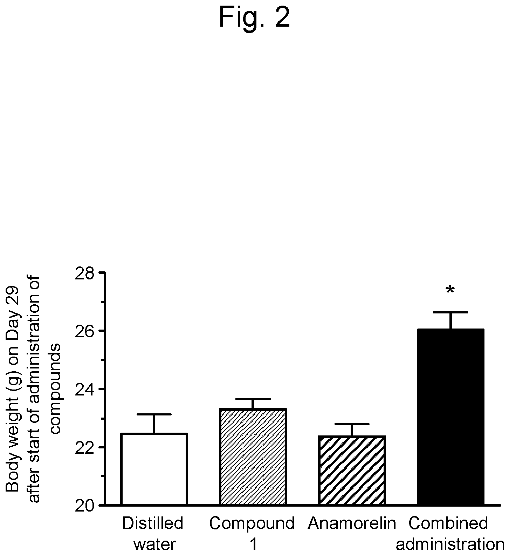

The results are shown in Figures 1 and 2. In Figure 1, the ordinate represents the cumulative food intake in the period of administration of the compounds (from the start of administration to the day next to the final day of administration). In Figure 2, the ordinate represents the body weight on Day 29 from the start of administration of the compounds (the day next to the final day of administration) (after 28-day administration). In the abscissas of Figures 1 and 2, "Distilled water" represents distilled water-treated group, "Compound 1" represents Compound 1-treated group, "Anamorelin" represents anamorelin hydrochloride-treated group, and "Combined administration" represents Compound 1 and anamorelin hydrochloride-combined treatment group. In Figures 1 and 2, the mark "^{∗}" represents a statistical significance (^{∗}: P < 0.05) in comparison to distilled water-treated group (Dunnett's multiple test).

For the cumulative food intake in the period of administration of the compounds, while any statistically significant increase of food intake was not observed in Compound 1-treated group or anamorelin hydrochloride treated-group compared to distilled water-treated group, statistically significant increase of food intake was exhibited in Compound 1 and anamorelin hydrochloride combined-treatment group compared to distilled water-treated group. For the body weight after the compounds were administered for 28 days, while any statistically significant increase in body weight was not observed in Compound 1-treated group or anamorelin hydrochloride-treated group compared to distilled water-treated group, statistically significant increase of body weight was exhibited in Compound 1and anamorelin hydrochloride-combined treatment group compared to distilled water-treated group.

In the results above, although anamorelin hydrochloride alone did not exhibit effectiveness to the food intake and the body weight, the effect was expressed by combined administration of Compound 1 and anamorelin hydrochloride. This suggests that the tumor-bearing model animals had ghrelin resistance, and combined use of Compound 1 and anamorelin hydrochloride improved cancer cachexia in ghrelin-resistant states.

### (Example 2) Effect of combined use of Compound 1 and anamorelin hydrochloride in tumor-bearing mouse model (non-small cell lung cancer model):

Using tumor-bearing model animals where human alveolar basement epidermal gland cancer cells, A549 cells, were planted to nude mice, examination was performed about the drug efficacy on the food intake and body weight when Compound 1 and anamorelin hydrochloride were combined.

Subculture of A549 cells was performed using a 10% FBS-containing RPMI1640 culture medium. In evaluation of drug efficacy, 7-week old female BALB/C slc/nu/nu mice (Japan SLC, Inc.) were purchased, and were used after habituated for one week. Cancer-bearing model animals were prepared as follows. Specifically, 2.5 × 10⁷ cells of A549 cells were planted to the right abdominal of each mouse by subdermal administration. On Day 22 from the cell plantation, the mice were divided into groups to have an equal averaged tumor volume.

The effects of a variety of compounds on food intake and body weight were examined as follows. Specifically, for 28 days from Day 23 to Day 50 from the cell plantation, the compounds were orally administered daily, and the food intake and the body weight were measured at the same time. For the doses of the compounds, the dose of Compound 1 was 0.25 mg/kg (13 examples), and the dose of anamorelin hydrochloride was 30 mg/kg (13 examples). In combined administration of Compound 1 and anamorelin hydrochloride, 0.25 mg/kg of Compound 1 and 30 mg/kg of anamorelin hydrochloride were used, where Compound 1 and anamorelin hydrochloride preliminarily dissolved in distilled water were mixed for preparation (13 examples). As a control, distilled water was administered in the same manner (13 examples). The food intake was calculated by subtracting the weight of the lid of each single cage containing food on the previous day from the weight of the lid of the single cage containing food on the day of measurement.

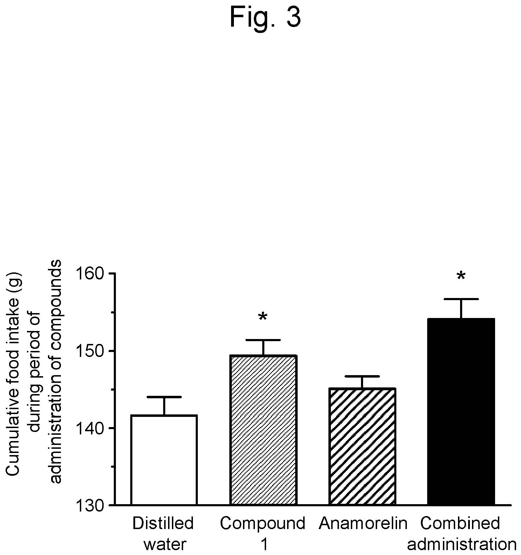

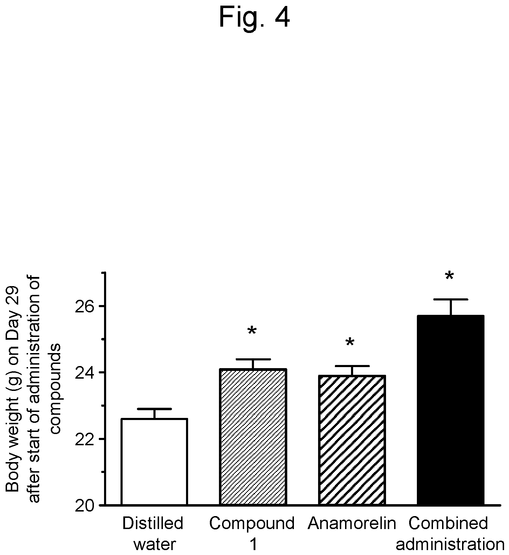

The results are shown in Figures 3 and 4. In Figure 3, the ordinate represents the cumulative food intake in the period of administration of the compounds (from the start of administration to the day next to the final day of administration). In Figure 4, the ordinate represents the body weight on Day 29 from the start of administration of the compounds (the day next to the final day of administration) (after 28-day administration). In the abscissas of Figures 3 and 4, "Distilled water" represents distilled water-treated group, "Compound 1" represents Compound 1-treated group, "Anamorelin" represents anamorelin hydrochloride-treated group, and "Combined administration" represents Compound 1 and anamorelin hydrochloride combined-treatment group. In Figures 3 and 4, the mark "^{∗}" represents a statistical significance (^{∗}: P < 0.05) in comparison to distilled water-treated group (Dunnett's multiple test).

For the cumulative food intake in the period of administration of the compounds, statistically significant increase of food intake was exhibited in Compound 1-treated group and Compound 1 and anamorelin hydrochloride-combined treatment group compared to distilled water-treated group, and a tendency of increasing food intake was observed in anamorelin hydrochloride-treated group compared to distilled water-treated group. The most remarkable increase of food intake was observed in Compound 1 and anamorelin hydrochloride-combined treatment group. For the body weight after the compounds were administered for 28 days, statistically significant increase of body weight was exhibited in all of Compound 1-treated group, anamorelin hydrochloride-treated group, and Compound 1 and anamorelin hydrochloride-combined treatment group compared to distilled water-treated group, and the most remarkably increase of body weight was observed in Compound 1 and anamorelin hydrochloride-combined treatment group.

### (Example 3) Effect of combined use of Compound 1 and anamorelin hydrochloride in tumor-bearing mouse model (skin cancer model):

Using tumor-bearing model animals where mouse malignant melanoma cells, B16/F10 cells, were planted to mice, and examination was performed about the drug efficacy to the food intake and the body weight when Compound 1 and anamorelin hydrochloride were combined.

Subculture of B16/F10 cells was performed using a DMEM culture medium containing 10% FBS and penicilin-streptomycin. In evaluation of the drug efficacy, 6-weeks old female C57BL/6J mice (Charles River Laboratories, Japan, Inc.) when introduced were used after habituated for one week. The tumor-bearing model animals were prepared as follows. Specifically, 5 × 10⁶ cells of B16/F10 cells were planted to the right abdominal of each mouse by subdermal administration. On Day 5 from the cell plantation, the mice were divided into groups to have an equal averaged tumor volume.

The effects of a variety of compounds on food intake and body weight were examined as follows. Specifically, for 11 days from Day 5 to Day 15 from the cell plantation, the compounds were orally administered daily, and the food intake and the body weight were measured at the same time. For the doses of the compounds, the dose of Compound 1 was 0.05 mg/kg (8 examples), and that of anamorelin hydrochloride was 30 mg/kg (8 examples). In combined administration of Compound 1 and anamorelin hydrochloride, 0.05 mg/kg of Compound 1 and 30 mg/kg of anamorelin hydrochloride were used, where Compound 1 and anamorelin hydrochloride preliminarily dissolved in distilled water were mixed for preparation (8 examples). As a control, distilled water was administered in the same manner (8 examples). The food intake was calculated by subtracting the weight of the lid of each single cage containing food on the previous day from the weight of the lid of the single cage containing food on the day of measurement. Eight individuals of each group were divided into two cases, in each of which 4 individuals were raised. The food intake per one mouse was calculated by dividing the total food intake of the 2 cases by 8.

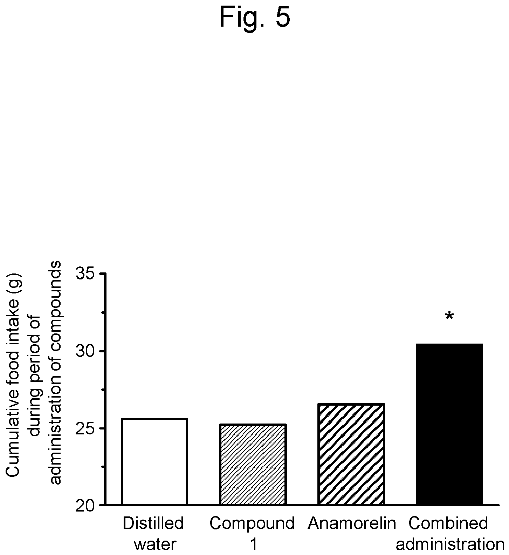

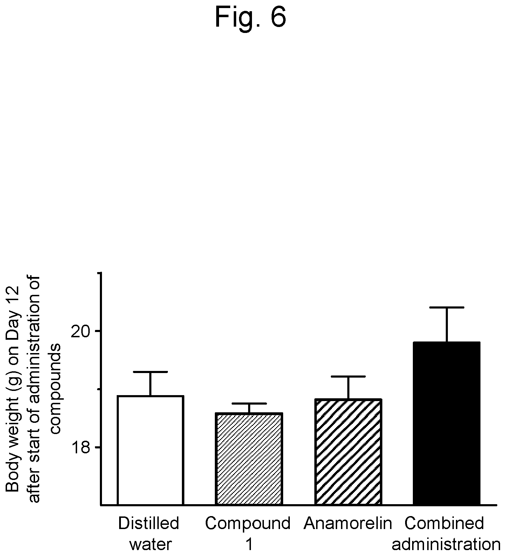

The results are shown in Figures 5 and 6. In Figure 5, the ordinate represents the cumulative food intake in the period of administration of the compounds (from the start of administration to the day next to the final day of administration). In Figure 6, the ordinate represents the body weight on Day 12 from the start of administration of the compounds (the day next to the final day of administration) (after 11-day administration). In the abscissas in Figures 5 and 6, "Distilled water" represents distilled water-treated group, "Compound 1" represents Compound 1-treated group, "Anamorelin" represents anamorelin hydrochloride-treated group, and "Combined administration" represents Compound 1 and anamorelin hydrochloride-combined treatment group. In Figure 5, the mark "^{∗}" represents a statistical significance (^{∗}: P < 0.05) in comparison to distilled water-treated group (Dunnett's multiple test).

For the cumulative food intake in the period of administration of the compounds, while any statistically significant increase food intake was not observed in Compound 1-treated group and anamorelin hydrochloride-treated group compared to distilled water treated group, statistically significant increase of food intake was exhibited in Compound land anamorelin hydrochloride-combined treatment group compared to distilled water-treated group. For the body weight after the compounds were administered for 11 days, a tendency of increasing body weight was not exhibited in all Compound 1-treated group, anamorelin hydrochloride-treated group, and Compound land anamorelin hydrochloride-combined treatment group compared to distilled water-treated group, the tendency of increasing body weight was exhibited in Compound 1 and anamorelin hydrochloride-combined treatment group.

The results above show that the increasing action of the food intake and the body weight by the combined administration of Compound 1 and anamorelin hydrochloride was observed not only in the non-small cell lung cancer model but also in the skin cancer model, thus showing that combined use of Compound 1 and anamorelin hydrochloride improves cancer cachexia caused by a variety of types of cancer.

The results have revealed that remarkable symptom suppressing effects to cachexia are exhibited by combined use of a compound represented by General Formula (I) or a pharmacologically acceptable acid addition salt thereof and a ghrelin receptor agonist in doses each of which does not result in demonstration of the food intake and body weight increasing action. It is also shown that combined use of the compound represented by General Formula (I) or a pharmacologically acceptable acid addition salt thereof and the ghrelin receptor agonist can reduce the dosage(s) of both or one of them, thereby reducing concerns on side effects. Furthermore, it is shown that this effect of combined use is an effect demonstrated in malignant tumors in general.

### INDUSTRIAL APPLICABILITY

The therapeutic or prophylactic agent for cachexia according to the present invention has a high therapeutic effect to cachexia, and thus is useful in the medical drug field.

All the publications, Patents, and Patent applications cited in this specification, as they are, are incorporated in this specification by reference.

## Claims

1. A therapeutic or prophylactic agent for cachexia wherein a compound represented by the following General Formula (I): 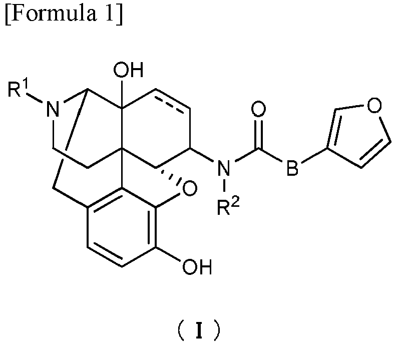
wherein a double line composed of a dotted line and a solid line represents a double bond or a single bond, R¹ represents a cycloalkylalkyl having 4 to 7 carbon atoms, R² represents a linear or branched alkyl having 1 to 5 carbon atoms, and B represents a-CH=CH-,
or a pharmacologically acceptable acid addition salt thereof and a ghrelin receptor agonist are used in combination.

2. A therapeutic or prophylactic agent for cachexia comprising a compound represented by the following General Formula (I): 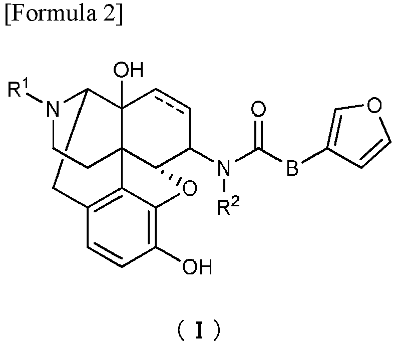
wherein a double line composed of a dotted line and a solid line represents a double bond or a single bond, R¹ represents a cycloalkylalkyl having 4 to 7 carbon atoms, R² represents a linear or branched alkyl having 1 to 5 carbon atoms, and B represents a -CH=CH-,
or a pharmacologically acceptable acid addition salt thereof and a ghrelin receptor agonist as active ingredients.

3. The therapeutic or prophylactic agent according to claim 1 or 2, wherein R¹ is a cyclopropylmethyl, a cyclobutylmethyl, a cyclopentylmethyl, or a cyclohexylmethyl, and R² is a methyl, an ethyl, or a propyl.

4. The therapeutic or prophylactic agent according to claim 1 or 2, wherein R¹ is a cyclopropylmethyl, R² is a methyl, and B is a trans-form -CH=CH-.

5. The therapeutic or prophylactic agent according to claim 1 or 2, wherein the compound represented by General Formula (I) is (-)-17-(cyclopropylmethyl)-3,14β-dihydroxy-4,5α-epoxy-6β-[N-methyl-trans-3-(3-furyl)acrylamide]morphinan represented by the structural formula:

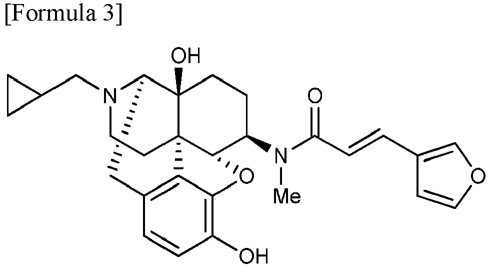

6. The therapeutic or prophylactic agent according to any one of claims 1 to 5, wherein the ghrelin receptor agonist is anamorelin or a pharmacologically acceptable salt thereof.

7. The therapeutic or prophylactic agent according to any one of claims 1 to 6, wherein the cachexia is cancer cachexia.
